# EUROPÄISCHE PATENTSCHRIFT

(11) **EP 0 663 197 B1**
(45) Veröffentlichungstag und Bekanntmachung des Hinweises auf die Patenterteilung: **30.12.1998**
(21) Anmeldenummer: 94100412.9
(22) Anmeldetag: 13.01.1994
(51) Int. Cl.: A61F 6/08, A61F 2/00

(54) **Aufblasbarer, ballonartiger Stützkörper**
Inflatable balloon-shaped supporting device
Dispositif de support gonflable en forme de ballon

(43) Veröffentlichungstag der Anmeldung: 19.07.1995
(73) Patentinhaber: Hammersen, Eckehard, Dr., D-37130 Diemarden (DE)
(72) Erfinder: Hammersen, Eckehard, Dr., D-37130 Diemarden (DE)
(74) Vertreter: Rehberg, Elmar, Dipl.-Ing.

(56) Entgegenhaltungen:
- DE-C- 82 551
- DE-C- 884 398
- US-A- 2 365 296
- US-A- 2 769 442
- US-A- 4 669 478

## Beschreibung

Die Erfindung bezieht sich auf einen aufblasbaren, ballonartigen Stützkörper auf gummielastischem Material zum Einführen in die Scheide mit den im Oberbegriff des Patentanspruchs 1 angegebenen Merkmalen. Der Stützkörper besteht aus körperverträglichem Material und dient dazu, bei z. B. einem Descesus, bei Uterus-prolaps oder bei Harninkontinenz, zur Besserung der Beschwerden in die Scheide eingeführt und aufgeblasen zu werden.

Aus der DE-A-37 00 239 ist ein aufblasbarer ballonartiger Stützkörper mit den im Oberbegriff des Patentanspruches 1 angegebenen Merkmalen bekannt. Der ballonartige Stützkörper weist runde Ballonform auf und geht nach unten in ein Schlauchstück über, in welchem ein Ventil angeordnet ist. Das Ventil ist als Rückschlagventil ausgebildet. Der ballonartige Stützkörper besitzt konstante Wandstärke und eine etwa kugelförmige Gestalt.

Aus der US - A - 2,365,296 ist auch ein aufblasbarer Stützkörper aus gummielastischem Material bekannt, der in etwa Birnenform aufweist. Es ist ein starrer Rohrabschnitt vorgesehen, der den Innenraum des aufblasbaren Stützkörpers durchsetzt und sowohl an einer Basisstelle als auch an einer zentralen Verdickung in der Deckfläche des birnenförmigen Stützkörpers mit diesem dichtend verbunden ist. Der Rohrabschnitt ist relativ kurz gestaltet, weist an seinem Ende ein Ventil auf und dient durch seine starre Ausbildung dem Einführen des Stützkörpers in die Scheide.

Aus der DE-C-38 00 744 ist ein weiterer aufblasbarer, ballonartiger Stützkörper bekannt. Der Stützkörper weist einen formsteifen und druckelastischen Grundkörper länglicher Form und gerundeter Spitze auf, der von einem dehnbaren Hüllkörper, der an dem Grundkörper anliegt, umschlossen ist. Im Basisbereich ist der Grundkörper mit dem Hüllkörper verbunden. Weiterhin ist im Basisbereich des Grundkörpers ein Ventil vorgesehen. Der Grundkörper besteht aus nicht-elastischem Material und ist von Luftführungskanälen durchsetzt, die an das Ventil angeschlossen sind. Das Ventil schließt mit der Basis des Grundkörpers bündig ab oder ist sogar gegenüber diesem versetzt angeordnet. An einem Ansatzstück des Ventils kann ein Blasebalg angeschlossen werden, mit dessen Hilfe Luft in den Zwischenraum zwischen dem Grundkörper und dem Hüllkörper einführbar ist, so daß sich der Hüllkörper ballonartig aufweitet und damit sich gegenüber dem Grundkörper beträchtlich vergrößert. Der Hüllkörper besitzt abgesehen von seinem Basisbereich eine konstante Wanddicke.

Der Erfindung liegt die Aufgabe zugrunde, einen aufblasbaren, ballonartigen Stützkörper der eingangs beschriebenen Art aufzuzeigen, der geeignet ist, z. B. bei einem Descensus, bei Uterus-prolaps oder bei manchen Formen der Harninkontinenz zur Linderung der Beschwerden in die Scheide eingesetzt zu werden, um dort die betreffenden Körperteile gegeneinander abzustützen.

Erfindungsgemäß wird dies bei einem aufblasbaren, ballonartigen Stützkörper der eingangs beschriebenen Art dadurch erreicht, daß der ballonartige Stützkörper abweichend von der runden Ballonform eine eingezogene Deckfläche mit ringförmiger Verstärkung aufweist und sich in Richtung von der Deckfläche auf das Schlauchstück konisch verjüngt, und daß der zentrale Bereich (12) der Deckfläche (4) konkav eingezogen ausgebildet ist.

Der ballonartige Stützkörper besitzt damit weder Kugelform, noch Birnenform, sondern er ist auf seiner dem Schlauchstück abgekehrten Seite eingezogen und bildet dort eine Deckfläche, die konkav ausgebildet ist. Die Deckfläche ist weiterhin weich und nachgiebig ausgebildet. Diese Deckfläche besitzt eine sie ringförmig umgebende Deckfläche, d.h. sie bildet gleichsam einen Reifen oder Ring für eine wirkungsvolle Abstützung in Verbindung mit der weichen und nachgiebigen Deckfläche, die an der ringförmigen Verstärkung aufgehängt ist. In Richtung auf das Schlauchstück verjüngt sich der ballonartige Stützkörper, wobei die Formgebung an dieser Stelle von untergeordneter Bedeutung ist. Der ballonartige Stützkörper ist damit nicht wie ein Luftballon mit konstanter, relativ dünner Wanddicke gestaltet, sondern er besitzt insbesondere über seine Länge unterschiedliche Wanddickenbereiche, durch die der Stützkörper im nicht-aufgeblasenen Zustand zusammengefaltet bzw. zusammengelegt werden kann, ähnlich einem Regenschirm, jedoch in aufgeblasenem Zustand eine weitgehend formstabile Gestalt einnimmt, die durch weiteres Lufteinblasen sich in ihrer Gestalt nur geringfügig verändert. Der Stützkörper weist damit die nötige Elastizität und durch die lokalen Verstärkungen auch die erforderliche Formstabilität auf. Die Form des aufgeblasenen Stützkörpers ist vergleichbar etwa der eines oben eingedellten Fesselballons und ist damit der Form- und Dehnbarkeit der Scheide entsprechend am oberen Ende deutlich weiter als im Basisbereich. In aufgeblasenem Zustand stellt der Stützkörper einen weitgehend stabilien Formkörper dar, der infolge seiner Konizität auch beim Anspannen der Bauchmuskulatur, z. B. beim Husten, Niesen u. dgl. nicht aus der Scheide herausgedrückt wird. Mit einer z. B. tellerähnlichen, rundlich bis quadratischen Bodenfläche oder auch im direkten Übergang ist ein Schlauchstück dichtend verbunden, welches je nach Länge entweder nur aus der Scheide heraushängt und die Betätigung des Ventils gestattet oder aber an der Unterwäsche mittels einer Klemme befestigt werden kann. Über das Ventil kann entweder durch Ansatz einer Pumpe oder eines Blasebalgs an das freie Ende des Schlauchstücks der Stützkörper aufgeblasen werden. In eingesetztem und nachfolgend aufgeblasenem Zustand stabilisiert der Stützkörper die vordere und hintere Scheidenwand, d. h. die vordere Scheidenwand und damit der Blasenboden wird ventro-cranial angehoben, die hintere Scheidenwand dorso-cranial. Gleichzeitig werden die seitlichen Scheidenwände nach latero-cranial gestreckt und der Uterus bzw. das obere Scheidenende angehoben. Der ballonartige Stützkörper kann stundenweise oder auch über den ganzen Tag getragen werden und erbringt eine Besserung der eingangs beschriebenen Beschwerden. Soll der Stützkörper aus der Scheide entfernt werden, so wird er nach dem Ablassen der Luft herausgezogen und ist nach Abwaschen und Trocknen jederzeit wiederverwendbar.

Der zentrale Bereich der Deckfläche ist zweckmäßig weich und nachgiebig ausgebildet, während die Randbereiche der Deckfläche relativ druckstabil ausgebildet sind. Damit kann sich die Deckfläche der individuellen Form des Muttermunds entsprechend anpassen.

Der ballonartige Stützkörper kann in den Querschnitten zu seiner Längsachse etwa rechteckige oder quadratische Form mit abgerundeten, ausgesteiften Kanten aufweisen. Es ist aber auch möglich, daß der Stützkörper rotationssymmetrisch zu seiner Längsachse ausgebildet ist und eine trichterartige Gestalt besitzt, die in die konkav eingezogene Deckfläche übergeht. Auch ein solcher Stützkörper läßt sich wie ein Regenschirm zusammenfalten und in die Scheide einführen.

Die ringförmige Verstärkung der Deckfläche kann durch eine vergleichsweise vergrößerte Wanddicke des Stützkörpers an dieser Stelle gebildet sein, wobei die Wanddicke an den verstärkten Stellen etwa 2 bis 5 mm betragen kann, während sie an den nichtverstärkten Stellen bis zu 2 mm dick sein kann.

Es ist aber auch möglich, die Aussteifungen und Verstärkungen durch Einlagen in den ballonartigen Stützkörper zu bilden. Als Einlagen können Kunststoffdrahtabschnitte, Gewebeeinlagen o. dgl. angewendet werden.

Der ballonartige Stützkörper kann eine Bodenfläche aufweisen. Die Bodenfläche und die Deckfläche können durch ein den Innenraum des Stützkörpers durchsetzendes Halteband miteinander verbunden sein. Dieses Halteband, welches aus dem Material des Stützkörpers bestehen kann, behindert einerseits das Zusammenfalten des Stützkörpers in nicht-aufgeblasenem Zustand nicht und sorgt andererseits dafür, daß sich der zentrale Bereich der Deckfläche, der in aller Regel dünnwandig ausgebildet ist, beim Aufblasen nicht nach außen durchwölbt, sondern in der nach innen leicht eingewölbten Form verbleibt und damit die Deckfläche stabilisiert.

Als Ventil kann vorzugsweise ein Rückschlagventil vorgesehen sein, wobei es zweckmäßig ist, z. B. solche Ventile zu verwenden, wie sie an Luftmatratzen oder Schwimmflügeln bekannt sind. In Verbindung damit kann auch das Rückschlagventil mit einem in das freie Ende des Schlauchstücks einsetzbaren Stöpsel versehen sein.

Es kann ein der Verlängerung dienender Schlauchabschnitt an das Schlauchstück dichtend ansetzbar sein, um das Aufblasen des Stützkörpers mit dem Mund in eingesetztem Zustand zu ermöglichen. Wie ersichtlich, kann die Patientin den Stützkörper selbständig einsetzen und handhaben, ohne daß es der Mithilfe eines Arztes bedarf.

Bevorzugte Ausführungsbeispiele des Stützkörpers werden im Folgenden beschrieben. Es zeigen:
- Figur 1: eine perspektivische Darstellung des Stützkörpers in aufgeblasenem Zustand,
- Figur 2: einen Längsschnitt durch den Stützkörper,
- Figur 3: einen Querschnitt durch den Stützkörper gemäß der Linie III-III in Figur 2,
- Figur 4: einen schematisierten Längsschnitt durch eine weitere Ausführungsform,
- Figur 5: einen Querschnitt gemäß der Linie V-V in Figur 4,
- Figur 6: einen schematischen Längsschnitt durch eine weitere Ausführungsform,
- Figur 7: einen schematischen Längsschnitt durch eine weitere Ausführungsform und
- Figur 8: einen Querschnitt durch den Stützkörper in Figur 7 gemäß der Linie VIII-VIII.

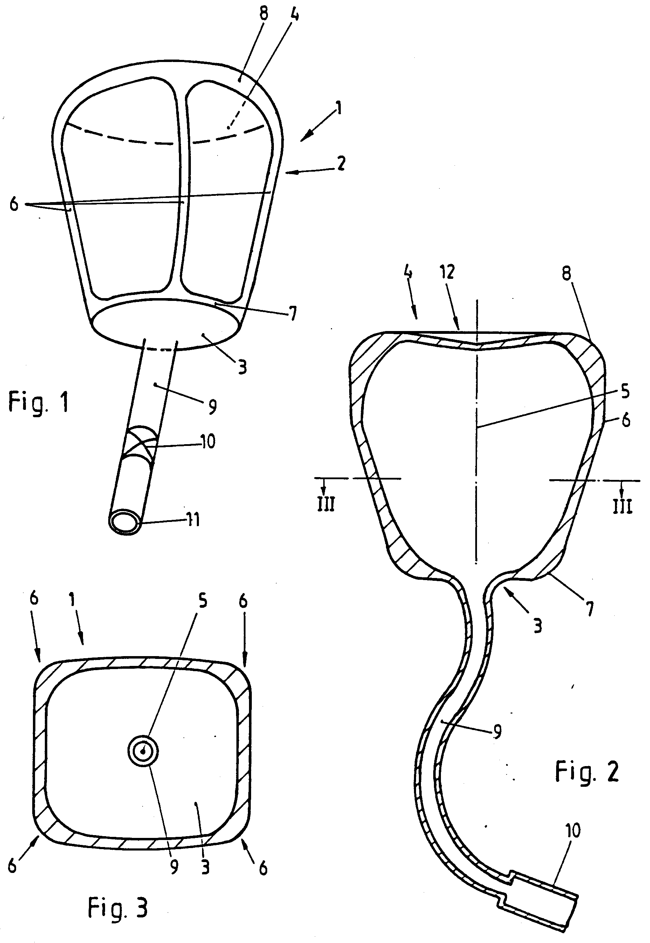

Der in Figur 1 schematisch dargestellte ballonartige Stützkörper 1 ist als Hohlkörper ausgebildet, weist einen konischen Mittelteil 2 auf, der im Basisbereich in eine Bodenfläche 3 übergeht, während am anderen Ende an den Mittelteil 2 eine Deckfläche 4 anschließt. Der Stützkörper 1 erweitert sich konisch von der Bodenfläche 3 in Richtung auf die Deckfläche 4, also in Richtung seiner Längsachse 5 (Figur 2).

Der Stützkörper 1 weist in seinem Mittelbereich ähnliche Querschnittsformen auf, wie es anhand von Figur 3 verdeutlicht ist. Der Stützkörper besteht aus gummielastischem Material, welches das Zusammenlegen des Stützkörpers parallel zu seiner Längsachse 5 gestattet, so daß er damit in eine stabähnliche, entsprechende Längsstabilität aufweisende Form gebracht werden kann, wie es für das Einführen in die Scheide zweckmäßig ist. Die Wanddicke des hohlen Stützkörpers 1 variiert, und zwar wird der Mittelteil 2 von abgerundeten, ausgesteiften Kanten 6 stabilisiert, in deren Bereich eine vergleichsweise größere Wanddicke vorliegt. Diese Kanten 6 gehen in eine ringförmige Verstärkung 7 im Bereich der Bodenfläche 3 und eine weitere ringförmige Verstärkung 8 im Bereich der Deckfläche 4 über. Die ringförmigen Verstärkungen 7 und 8 sind in sich geschlossen, müssen aber nicht unbedingt kreisförmige Gestalt aufweisen. Diese Zusammenlegbarkeit des Stützkörpers kann durch Schwachstellen im Bereich der Verstärkungen 7 und 8 begünstigt sein. Die Kanten 6 und Verstärkungen 7, 8 dienen dazu, in aufgeblasenem Zustand eine definierte Form des Stützkörpers herbeizuführen, wie sie aus den Figuren 1 bis 3 ersichtlich ist. Selbstverständlich ist es möglich, den Stützkörper 1 in verschiedenen Größen- bzw. Längenabstufungen herzustellen, um unterschiedlichen Geometrien im Bereich der Scheide Rechnung zu tragen.

Mit der Bodenfläche 3 steht ein Schlauchstück 9 in Verbindung, in dessen Bereich ein hier nur schematisch angedeutetes Ventil 10 angeordnet ist. Das Schlauchstück 9 kann auch einstückig aus dem Material des Stützkörpers ausgeformt sein. Das Schlauchstück 9 besitzt im allgemeinen eine solche Länge, wie es für die Handhabung des Ventils 10 und des Stützkörpers 1 erforderlich ist. An das freie Ende 11 des Schlauchstücks 10 läßt sich ein der Verlängerung dienender Schlauchabschnitt anschließen (nicht dargestellt), der dazu dient, den eingeführten Stützkörper mit dem Mund aufzublasen. Es ist aber auch möglich, an das freie Ende 11 des Schlauchstücks 9 einen Blasebalg anzuschließen, um auf diese Art und Weise dem eingesetzten Stützkörper in den aufgeblasenen Zustand zu überführen. Wie aus Figur 2 ersichtlich ist, kann das Ventil 10 auch am freien Ende des Schlauchstücks 9 angeordnet sein.

Die in Figur 3 verdeutlichte Querschnittsform des Mittelteils 2 des Stützkörpers 1 kann am besten mit einer rechteckigen oder quadratischen Form beschrieben werden, wobei die ausgesteiften Kanten abgerundet ausgebildet sind. Im Bereich der Deckfläche 4 schließt die Verstärkung 8 einen zentralen Bereich 12 ein, der dünnwandig und weich ausgebildet ist. Es ist erkennbar, daß dieser zentrale Bereich 12 etwas nach innen eingeformt ist; zumindest wird diese Formgebung in eingesetztem, aufgeblasenem Zustand erreicht.

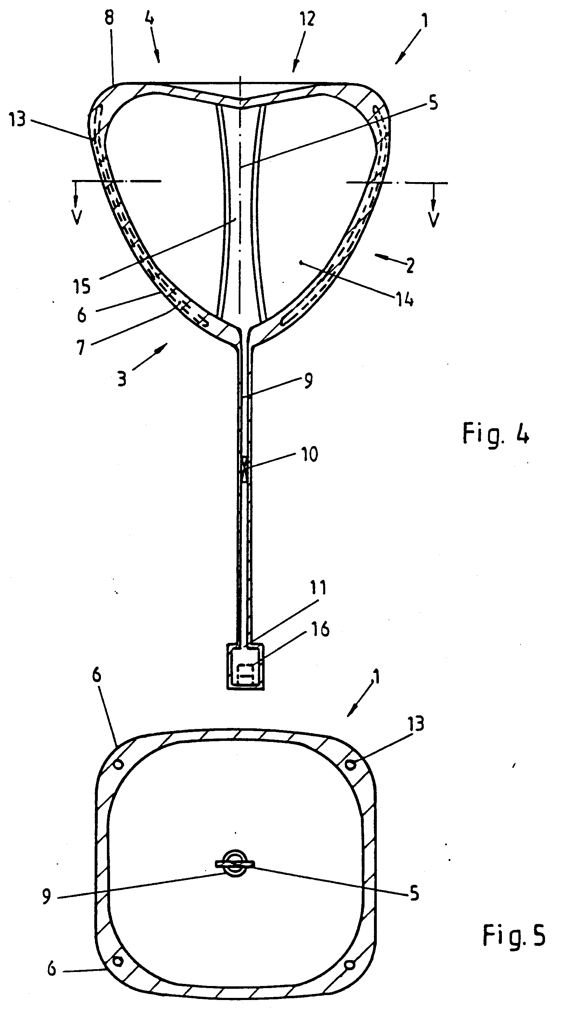

Bei der Ausführungform des Stützkörpers 1 gemäß den Figuren 4 und 5 ist die besondere Formgebung verwirklicht, die auch bereits anhand des Ausführungbeispiels der Figuren 1 bis 3 beschrieben wurde. Die Kanten 6 und die Verstärkungen 7 und 8 können hier durch Einlagen 13 aus Kunststoffdrahtabschnitten, Gewebeverstärkungen o. dgl. gebildet sein, die eine Längsstabilität erzeugen und andererseits ein unkontrolliertes Aufblasen mit einer nicht definierten Form, ähnlich einem Luftballon, verhindern. Zusätzlich ist der Innenraum 14 des Stützkörpers 1 etwa im Bereich der Längsachse 5 von einem Halteband 15 durchsetzt, welches die Bodenfläche 3 mit der Deckfläche 4 bzw. deren zentralem Bereich 12 verbindet. Damit wird die konkave Form der Deckfläche 4 zusätzlich stabilisiert. Andererseits behindert dieses Halteband 15 das Zusammenfalten des Stützkörpers 1 in entlüftetem Zustand nicht.

Das Ventil 10 ist auch im Mittelbereich des Schlauchstücks 9 vorgesehen. Am freien Ende 11 des Schlauchstücks 9 befindet sich ein Anschluß 16 für einen Blasebalg bzw. einen der Verlängerung dienenden Schlauchabschnitt.

Wie Figur 5 erkennen läßt, besitzt der Mittelteil 2 des Stützkörpers 1 bei dieser Ausführungsform etwa quadratischen Querschnitt mit abgerundeten Kanten 6.

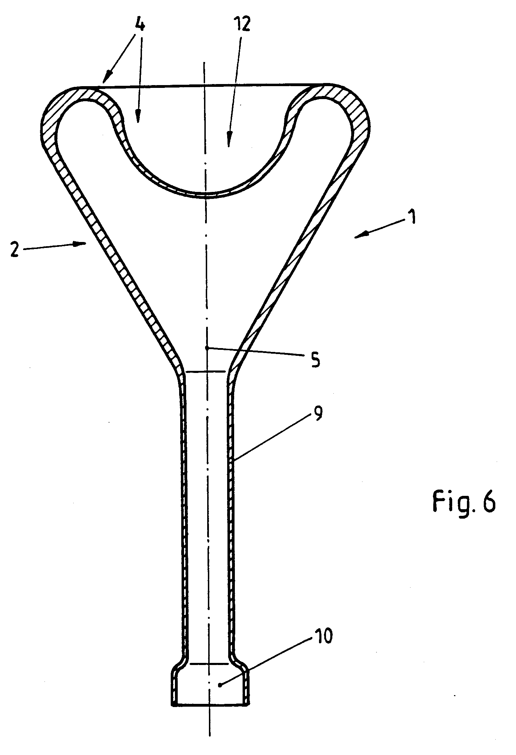

Figur 6 zeigt eine weitere Ausführungsform des ballonartigen Stützkörpers 1. Das Mittelteil 2 ist hier trichterartig ausgebildet und geht im oberen Bereich in eine Deckfläche 4 über, die ring- oder reifenförmige Gestalt besitzt. Der Stützkörper 1 ist rotationssymmetrisch zu seiner Längsachse 5 ausgebildet. Es fehlen verstärkende Kanten 6 sowie eine ausgeprägte Bodenfläche 3. Stattdessen geht der Stützkörper 1 an seinem der Deckfläche 4 abgekehrten Ende unmittelbar in das Schlauchstück 9 über, welches auch hier mit einem Ventil 10 ausgestattet ist, das nur angedeutet dargestellt ist. Aus Figur 6 ist gut zu erkennen, daß es auf die besondere Formgebung des ballonartigen Stützkörpers 1 im Bereich der Deckfläche 4 ankommt. Die Deckfläche 4 ist nach innen konkav eingezogen, und zwar mehr als dies bei den Ausführungsbeispielen der Figuren 2 und 4 in Erscheinung tritt. Zusätzlich ist die Wandstärke des Stützkörpers 1 im Außenbereich der Deckfläche 4 erheblich dicker als im Mittelbereich. Der Mittelbereich ist damit weich und nachgiebig gestaltet, während der äußere Bereich der Deckfläche 4 gleichsam einen stützenden Ring oder Reifen bildet. Die Formstabilität in aufgeblasenem Zustand ergibt sich insbesondere aus dieser Querschnittsgestaltung.

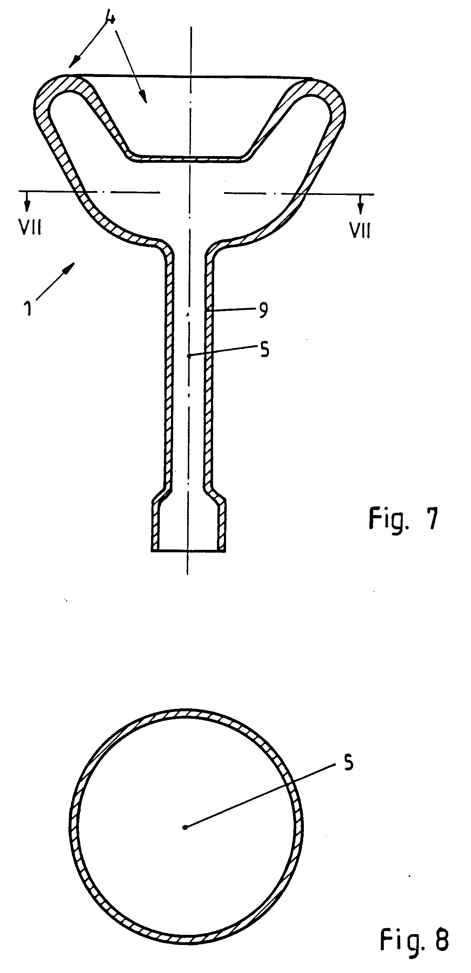

Das in den Figuren 7 und 8 dargestellte Ausführungsbeispiel des ballonartigen Stützkörpers 1 ist ebenfalls rotationssymmetrisch ausgebildet. Die Deckfläche 4 besitzt ringförmige Gestalt mit außen verdickter Wandstärke. Der Mittelteil der Deckfläche ist hier eben und dünnwandig gestaltet. Auch hier verjüngt sich der Stützkörper 1 von der Deckfläche 4 in Richtung auf das Schlauchstück 9. Die Verjüngung verläuft etwas konisch eingezogen, wie dies aus Figur 7 ersichtlich ist. Auch dieser Stützkörper 1 ist in aufgeblasenem Zustand besonders formstabil. Er kann andererseits in entlüftetem Zustand zusammengefaltet werden.

Figur 8 zeigt die rotationssymmetrische Ausbildung um die Längsachse 5.

## Patentansprüche

1. Aufblasbarer, ballonartiger Stützkörper (1) aus gummielastischem Material zum Einführen in die Scheide, wobei der ballonartige Stützkörper (1) sich von einem Basisbereich nach oben erweitert und in dem Basisbereich mit einem Schlauchstück (9) dichtend verbunden ist, in dem ein Ventil (10) angeordnet ist, **dadurch gekennzeichnet**, daß der ballonartige Stützkörper (1) abweichend von der runden Ballonform eine eingezogene Deckfläche (4) mit ringförmiger Verstärkung (8) aufweist und sich in Richtung von der Deckfläche (4) auf das Schlauchstück (9) konisch verjüngt, und daß der zentrale Bereich (12) der Deckfläche (4) konkav eingezogen ausgebildet ist.

2. Stützkörper nach Anspruch 1, **dadurch gekennzeichnet**, daß der zentrale Bereich (12) der Deckfläche (4) weich und nachgiebig ausgebildet ist.

3. Stützkörper nach Anspruch 1, **dadurch gekennzeichnet**, daß der ballonartige Stützkörper (1) in den Querschnitten zu seiner Längsachse (5) etwa rechteckige oder quadratische Form mit abgerundeten, ausgesteiften Kanten (6) aufweist.

4. Stützkörper nach Anspruch 1, **dadurch gekennzeichnet**, daß der Stützkörper (1) rotationssymmetrisch zu seiner Längsachse ausgebildet ist und eine trichterartige Gestalt besitzt, die in die konkav eingezogene Deckfläche (4) übergeht.

5. Stützkörper nach einem der Ansprüche 1 bis 4, **dadurch gekennzeichnet**, daß die ringförmige Verstärkung (8) der Deckfläche (4) durch eine vergleichsweise vergrößerte Wanddicke des Stützkörpers (1) an dieser Stelle gebildet ist.

6. Stützkörper nach einem der Ansprüche 1 bis 5, **dadurch gekennzeichnet**, daß in dem ballonartigen Stützkörper (1) Einlagen (13) vorgesehen sind.

7. Stützkörper nach einem der Ansprüche 1 bis 6, **dadurch gekennzeichnet**, daß der ballonartige Stützkörper (1) eine Bodenfläche (3) aufweist und die Bodenfläche (3) und die Deckfläche (4) durch ein den Innenraum (14) des Stützkörpers (1) durchsetzendes Halteband (15) miteinander verbunden sind.

8. Stützkörper nach Anspruch 1, **dadurch gekennzeichnet**, daß als Ventil (10) ein Rückschlagventil vorgesehen ist.

9. Stützkörper nach Anspruch 1 und 8, **dadurch gekennzeichnet**, daß das Rückschlagventil mit einem in das freie Ende (11) des Schlauchstücks (9) einsetzbaren Stöpsel versehen ist.

10. Stützkörper nach Anspruch 1 oder 8, **dadurch gekennzeichnet**, daß ein der Verlängerung dienender Schlauchabschnitt an das Schlauchstück (9) dichtend ansetzbar ist.

## Claims

1. An inflatable, balloon-shaped support device (1) of rubber material for introduction into the vagina, wherein the balloon-shaped support device (1) enlarges upwardly from a base region and in the base region is sealingly connected to a hose portion (9) in which a valve (10) is arranged, characterised in that the balloon-shaped support device (1), as a departure from the round balloon shape, has an inwardly drawn top surface (4) with annular reinforcement (8) and conically tapers in a direction from the top surface (4) towards the hose portion (9), and that the central region (12) of the top surface (4) is of a concavely inwardly drawn configuration.

2. A support device according to claim 1 characterised in that the central region (12) of the top surface (4) is of a soft and flexible nature.

3. A support device according to claim 1 characterised in that the balloon-shaped support device (1) is of approximately rectangular or square shape with rounded-off stiffened edges (6) in the cross-sections relative to its longitudinal axis (5).

4. A support device according to claim 1 characterised in that the support device (1) is rotationally symmetrical relative to its longitudinal axis and is of a funnel-like configuration which goes into the concavely inwardly drawn top surface (4).

5. A support device according to one of claims 1 to 4 characterised in that the annular reinforcement (8) of the top surface (4) is formed by a comparatively enlarged wall thickness of the support device (1) at that location.

6. A support device according to one of claims 1 to 5 characterised in that inserts (13) are provided in the balloon-shaped support device (1).

7. A support device according to one of claims 1 to 6 characterised in that the balloon-shaped support device (1) has a bottom surface (3) and the bottom surface (3) and the top surface (4) are joined together by a holding band (15) which passes through the interior (14) of the support device (1).

8. A support device according to claim 1 characterized in that a check valve is provided as the valve (10).

9. A support device according to claim 1 and claim 8 characterised in that the check valve is provided with a stopper which can be inserted into the free end (11) of the hose portion (9).

10. A support device according to claim 1 or claim 8 characterised in that a hose part which serves for extension purposes can be sealingly attached to the hose portion (9).

## Revendications

1. Corps de soutien (1) du type ballon gonflable réalisé dans un matériau présentant l'élasticité du caoutchouc destiné à être introduit dans le vagin, le corps de soutien (1) du type ballon s'élargissant vers le haut à partir d'une zone de base et état assemblé de manière étanche dans la zone de base avec un morceau de tuyau (9), dans lequel est disposeé une soupape (10), caractérisé en ce que le corps de soutien (1) du type ballon présente, à la différence d'une forme ronde de ballon, une surface supérieure (4) en retrait avec renfort (8) annulaire et se rétrécit coniquement depuis la surface supérieure (4) vers le morceau de tuyau (9), et en ce que la zone centrale (12) de la surface supérieure (4) est concave.

2. Corps de soutien selon la revendication 1, caractérisé en ce que la zone centrale (12) de la surface supérieure (4) est souple et élastique.

3. Corps de soutien selon la revendication 1, caractérisé en ce que le corps de soutien (1) du type ballon présente dans les sections trasversales à son axe longitudinal (5), une forme à peu près rectangulaire ou carrée avec des angles (6) raidis et arrondis.

4. Corps de soutien selon la revendication 1, caractérisé en ce que le corps de soutien (1) présente une symétrie de révolution par rapport à son axe longitudinal et possède une forme du type entonnoir, qui se prolonge par la surface supérieure (4) concave.

5. Corps de soutien selon l'une des revendications 1 à 4, caractérisé en ce que le renfort (8) annulaire de la surface supérieure (4) est constitué à cet endroit par une épaisseur de paroi relativement augmentée du corps de soutien (1).

6. Corps de soutien selon l'une des revendications 1 à 5, caractérisé en ce que des garnitures (13) sont prévues dans le corps de soutien (1) du type ballon.

7. Corps de soutien selon l'une des revendications 1 à 6, caractérisé en ce que le corps de soutien (1) du type ballon présente une surface inférieure (3), et en ce que la surface inférieure (3) et la surface supérieure (4) sont assemblées entre elles par une bande de maintien (15) traversant le volume intérieur (14) du corps de soutien (1).

8. Corps de soutien selon la revendication 1, caractérisé en ce qu'un clapet de non-retour est prévu en tant que soupape (10).

9. Corps de soutien selon les revendications 1 et 8, caractérisé en ce que le clapet de non-retour est pourvu d'un bouchon à insérer dans l'extrémité libre (11) du morceau de tuyau (9).

10. Corps de soutien selon la revendication 1 ou 8, caractérisé en ce qu'un tronçon de tuyau servant de rallonge peut être placé de manière étanche sur le morceau de tuyau (9).
